**Europäisches Patentamt**

**European Patent Office**

**Office européen des brevets**

(19)

(11)  **EP 0 550 703 B1**

(12)  **EUROPÄISCHE PATENTSCHRIFT**

(45) Veröffentlichungstag und Bekanntmachung des Hinweises auf die Patenterteilung:
**24.11.1999 Patentblatt 1999/47**

(21) Anmeldenummer: 92907782.4

(22) Anmeldetag: 03.04.1992

(51) Int. Cl.$^6$: **A61K 35/78**

(86) Internationale Anmeldenummer:
**PCT/EP92/00751**

(87) Internationale Veröffentlichungsnummer:
**WO 93/02688 (18.02.1993 Gazette 1993/05)**

(54) **ARZNEIMITTEL AUF PFLANZLICHER BASIS ZUR TONUSERHÖHUNG UND ZUR TONUSMODULIERUNG DER GLATTMUSKULÄREN ORGANE**

PLANT-BASED MEDICAMENT FOR ENHANCING AND CONTROLLING THE TONE OF SMOOTH-MUSCLE ORGANS

MEDICAMENT A BASE DE PLANTES POUR AUGMENTER ET MODULER LE TONUS DES ORGANES MUSCULAIRES LISSES

(84) Benannte Vertragsstaaten:
**AT BE CH DE DK ES FR GB GR IT LI LU MC NL SE**

(30) Priorität: **29.07.1991 DE 4125024**

(43) Veröffentlichungstag der Anmeldung:
**14.07.1993 Patentblatt 1993/28**

(73) Patentinhaber:
**STEIGERWALD ARZNEIMITTELWERK GMBH D-64295 Darmstadt (DE)**

(72) Erfinder: **OKPANYI, Samuel, N.**
**D-6200 Wiesbaden (DE)**

(74) Vertreter:
**Luderschmidt, Schüler & Partner GbR Patentanwälte,
Postfach 3929
65029 Wiesbaden (DE)**

(56) Entgegenhaltungen:
• **BUNDESVERBAND DER PHARMAZEUTISCHEN INDUSTRIE E.V. 'ROTE LISTE 1990' 1990 , EDITIO CANTOR , AULENDORF 59 184 IBEROGAST**
• **Heilpflanzen-Lexicon für Ärzte und Apotheker, Gustav Fischer Verlag 1981, S.122.**

EP 0 550 703 B1

**Beschreibung**

Die Erfindung bezieht sich auf ein Arzneimittel auf pflanzlicher Basis mit tonisierender Wirkung auf atonische bzw. tonusgeminderte, glattmuskuläre Organe. Weiterhin bezieht sie sich auf ein solches Arzneimittel mit tonusmodulierender Wirkung auf tonusgeminderte oder tonusgesteigerte derartige Organe.

Bei den gastroenterologischen Diagnosen wie Dyspepsie, funktionelle gastrointestinale Beschwerden, Reizmagen, Reizdarm, mit vielschichtigen und vielfältigen Symptomen, steht die tonusbedingte Fehlregulation der Magen-Darm-Motorik bzw. -Motilität im Vordergrund. Es treten sowohl Krämpfe (Spasmen) als auch hypotone Opstipation (Atonie) mit verminderter Magen-Darm-Entleerung auf, begleitet durch solche Störungen, wie Druck und Schmerz im Oberbauch, Gastrokardialschmerz, Aufstoßen, Sodbrennen, Völlegefühl, Ösophagitis sowie Gastritis bis hin zu Ulzera im Magen-Darm.

In der Therapie kennt man vorwiegend solche chemische Arzneimittel, die entweder spasmolytisch anticholinergisch wirken oder die nebenwirkungsbehafteten chemischen Prokinetika und Dopaminantagonisten. Auch ein Arzneimittel auf pflanzlicher Basis ist bekannt. An Pflanzenbestandteilen weist es zwingend eine Mischung aus alkoholischem Frischpflanzenauszug Iberis amara totalis, zusammen mit alkoholischen Drogenauszügen von Angelicae radix, Cardui mariae fructus, Carvi fructus, Chelidonii herba, Liquiritae radix, Matricariae flos, Melissae folium, Menthae piperitae folium auf. Es liegt somit eine relativ große Anzahl von Wirkstoffen vor, die der Patient einzunehmen gezwungen ist.

Die Aufgabe der Erfindung besteht darin, ein Arzneimittel auf pflanzlicher Basis mit einer begrenzten Anzahl von Wirk-/Inhaltsstoffen, insbesondere Extrakten (Auszügen) zu schaffen, welches grundsätzlich tonuserhöhend bei erschlaffter glatter Muskulatur, insbesondere des Darmes, eingreift.

Diese Aufgabe ist gemäß der Erfindung dadurch gelöst, daß das Arzneimittel Iberis amara, vorzugsweise Iberis amara totalis (Blüten, Samen, Blätter, Stengel und Wurzeln), insbesondere als Pflanzenauszug als alleinigen Träger der Wirksamkeit enthält. Es hat sich überraschend gezeigt daß die Wirkstoffe dieser Pflanze ohne jegliche weitere Unterstützung und Wirkstoffe sowohl in prophylaktischer als auch therapeutischer Hinsicht zur Tonus- und Motilitätssteigerung bei hypotonen, glattmuskulären Organen, wie Magen-Darm-Trakt, Gallenblase, Harnblase, Venen und Uterus ausreicht. Die bisher zur Erzielung dieser Wirkung für erforderlich gehaltenen zusätzlichen Pflanzenwirkstoffe sind somit entbehrlich, und es kommt somit zu einer wesentlich geringeren Belastung des Patienten als bei der Einnahme der bekannten Arzneimittel.

In Weiterführung der Erfindung wurde auch überraschenderweise gefunden, daß ein Arzneimittel auf pflanzlicher Basis mit tonusmodulierender Wirkung auf tonusgeminderte oder tonusgesteigerte, glattmuskuläre Organe dadurch geschaffen wird, wenn es neben Iberis amara, vorzugsweise Iberis amara totalis (Blüten, Samen, Blätter, Stengel und Wurzeln), insbesondere als Pflanzenauszug, einen oder mehrere weitere Bestandteile der Gruppe, bestehend aus Menthae piperitae folium, Matricariae flos, Carvi fructus, Melissae folium und Liquiritiae radix, vorzugsweise als Pflanzenauszug, als alleinige Träger der Wirksamkeit enthält. Dieses erfindungsgemäße Arzneimittel zeichnet sich gegenüber dem vorstehend erwähnten bekannten Arzneimittel auf pflanzlicher Basis dadurch aus, daß es demgegenüber eine verminderte Anzahl von Pflanzenstoffen aufweist. Nicht vorhanden sind gegenüber den bekannten Arzneimittel Chelidonii herba, Cardui maria fructus und Angelicae radix. Es kann somit auf eine ganze Reihe u.U. belastender Stoffe bei der Behandlung von Patienten verzichtet werden. Trotzdem ist das erfindungsgemäße Arzneimittel in prophylaktischer und therapeutischer Hinsicht zur Tonus- und Motilitätsmodulierung bzw. -regulierung bei Erkrankungen von glattmuskulären Organen, wie Magen-Darm-Trakt, Gallenblase, Harnblase, Venen und Uterus voll wirksam, welche Erkrankungen kausal oder zur folge Tonus- und Motilitätsstörungen verschiedener Genese aufweisen. Die neben Iberis amara totalis bei dem erfindungsgemäßen Arzneimittel zur Anwendung gelangenden Pflanzen bzw. deren Wirkstoffe wirken vor allen Dingen spasmolytisch und erbringen im Zusammenwirken mit Iberis amara vollständig den angestrebten Effekt der tonusmodulierenden Wirkung auf tonusgeminderte oder tonusgesteigerte, glattmuskuläre Organe. Das erfindungsgemäße Arzneimittel kann sowohl als ein primäres (kausales) oder sekundäres Therapeutikum, insbesondere bei Beschwerden des Magen-Darm-Traktes mit den erwähnten Tonus- und Motilitätsstörungen sowohl bei hypo- als auch bei hypertonischen und -kinetischen Zuständen Einsatz finden.

Gemäß der Erfindung ist weiterhin vorgesehen, daß das Arzneimittel in Form von Pflanzenauszügen hergestellt wird und im wesentlichen besteht aus

15 - 40 Vol.-% Iberis amara,
10 - 30 Vol.-% Menthae piperitae,
20 - 40 Vol.-% Matricariae,
10 - 30 Vol.-% Carvi fructus,
10 - 30 Vol.-% Melissae folium,
10 - 30 Vol.-% Liquiritiae radix.

Ein besonders bevorzugtes Ausführungsbeispiel der Erfindung zeichnet sich dadurch aus, daß die Pflanzenauszüge wie folgt vorgesehen sind:

15 Vol.-% Iberis amara totalis,
10 Vol.-% Menthae piperitae folium,
30 Vol.-% Matricariae flos,
20 Vol.-% Carvi fructus,
15 Vol.-% Melissae folium,
10 Vol.-% Liquiritiae radix.

Im Rahmen der Erfindung liegt es auch, wenn der Pflanzenauszug, vorzugsweise bei Iberis amara, ein Frischpflanzenauszug ist. Hierdurch wird eine Reihe von Wirkstoffen dem Arzneimittel zugeführt, die bei der Herstellung eines Drogenauszugs u.U. nicht mehr voll vorhanden und somit nicht mehr wirksam werden könnten. Auch bei den weiteren in erfindungsgemäßer Weise in verringerter Anzahl gegenüber dem Stand der Technik vorhandenen Pflanzen können Frischpflanzenauszüge hergestellt werden, wenngleich in der Regel von diesen Pflanzen Drogenauszüge bereitgestellt werden.

Erfindungsgemäß beträgt bei dem Frischpflanzenauszug das Verhältnis von mazerierter oder perkolierter Pflanze zum Extrakt etwa 3 bis 7, vorzugsweise 6 Gramm Iberis amara : 10 Gramm. Bei dem Drogenauszug ist erfindungsgemäß ein Verhältnis von Drogen zum Extrakt, von etwa 1,5 bis 10, vorzugsweise 3,5 Gramm : 10 Gramm vorgesehen. Gemäß der Erfindung wird vorzugsweise als Extraktionsmittel für die Auszüge Ethanol in einer Endkonzentration von 30 bis 40 Vol.-%, Rest $H_2O$, verwendet..

Zusammenfassend läßt sich festhalten, daß überraschend gefunden wurde, daß das erfindungsgemäße Arzneimittel nach Anspruch 1 sowie nach Anspruch 2 bis 4 genauso wirksam wie und in besonderer Hinsicht sogar wirksamer als das bislang auf dem Markt befindliche Arzneimittel (STW 5) ist, welches - gegenüber dem erfindungsgemäßen Arzneimittel nach Anspruch 2 bis 4 -zusätzlich Chelidonii herba, Cardui mariae fructus und Angelicae radix enthält.

Bei den funktionellen Magen-Darm-Beschwerden handelt es sich um ein multifaktorielles Geschehen, bei dem das erfindungsgemäße Arzneimittel multifokal eingreift und dementsprechend, wie unten weiter ausgeführt, geprüft wurde.

Weitere Einzelheiten, Merkmale und Vorteile der Erfindung ergeben sich aus der folgenden Beschreibung, den Patentansprüchen sowie der Zeichnung. Es zeigen:

Fig. 1           den Einfluß des erfindungsgemäßen Arzneimittels nach Anspruch 1 auf den Tonus des ruhenden isolierten Meerschweinchen-Ileums;

Fig. 2           die grafische Wiedergabe der Tabelle 2;

Fig. 3           die grafische Wiedergabe der Tabelle 3;

Fig. 4 bis 7    den Einfluß des erfindungsgemäßen Arzneimittels nach Anspruch 2 sowie einen Vergleich des Einflusses des erfindungsgemäßen Arzneimittels nach Anspruch 2 gegenüber dem bekannten Arzneimittel auf pflanzlicher Basis (STW 5) auf die Histamin-induzierte Kontraktion (Spasmen) des Meerschweinchen-Ileums in den angegebenen unterschiedlichen Konzentrationen.

Tonuserhöhung

Der Einfluß von Iberis amara Frischpflanzenauszug gemäß Anspruch 1, im folgenden STW 6 genannt, auf den Tonus des Darmes wurde mit dem klassischen pharmakologischen Meerschweinchen-Ileum-Modell nach Magnus, R.: Versuche am überlebenden Dünndarm von Säugetieren, I. Mitteilung, Pflügers Archiv Ges. Physiol. 102, 123-151 (1904), sowohl am ruhenden als auch am mit Acetylcholin stimulierten Ileumstück im 37° C thermostatierten Organbad mit Krebs-Henseleit-Basislösung unter Carbogen-Begasung geprüft.

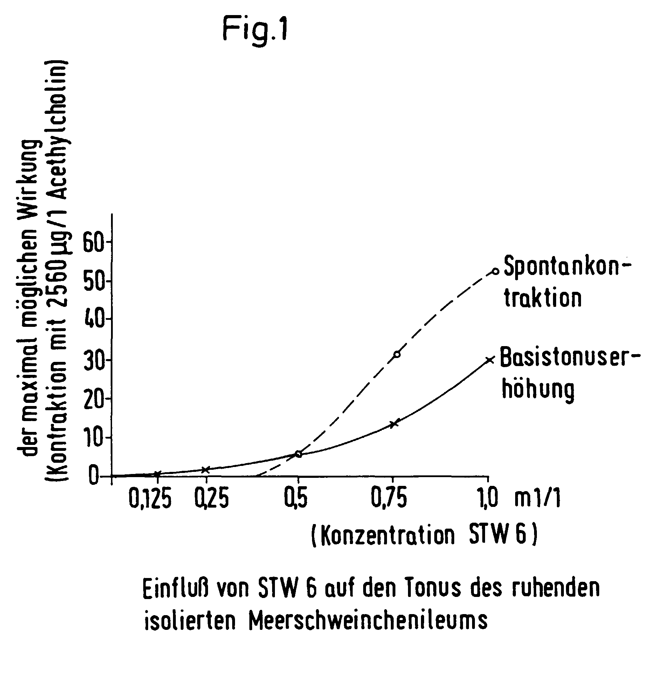

STW 6 führte bei dem ruhenden/erschlafften (atonischen) Meerschweinchenileum zu einer dosisabhängigen Basistonuserhöhung (Fig. 1), zusätzlich zu einer vollreversiblen Spontankontraktion.

Auf einen mit nur niedriger Acetylcholin-Konzentration (2,5 - 160 µg/l) stimulierten Darm (Hypotonus) bewirkt STW 6 ebenfalls eine dosisabhängige Tonussteigerung. Bei höheren Acetylcholin-Dosen (> 160 - 640 µg/l), bei denen hinreichend Tonus und Kontraktion vorherrschen, wirkt STW 6 nicht mehr tonuserhöhend.

Motilität des Magen-Darm-Traktes (Frischpflanzenauszug)

Weiterhin wurden Untersuchungen zum Einfluß von Iberis amara Frischpflanzenauszug auf die Motilität des Magen-Darmtraktes an Minipigs von Alaska-Hoke (Körpergewicht um 20 - 25 kg) durchgeführt. Unter Nembutal-Narkose wurden die Tiere endoskopiert und die Prüfsubstanzen unter endoskopischer Sicht auf die Magenschleimhaut aufgetragen.

Die Motilität wurde mittels Sonde und Zeitgeber bestimmt. Zuzüglich wurden folgende Parameter bestimmt: Azidität, nach Lanza, F.C.; Aspinall, R.L.; Swabb, E.A. et al.: A doubleblind, placebo-controlled, endoscopic comparison of the cytoprotective effects of misoprostol and cimetidine in tolmetin-induced gastric mucosal injury. Clinical development of misoprostol: Peptic ulcer disease and NSAID induced gastropathy, Chicago, May 1987 und Davenport (1964) Gewebshistamine, nach Lorenz, W.; Reimann, H.-J. et al.: A sensitive and specific method for the determination of histamine in human whole blood and plasma. Hoppe-Seyler's Z. Physiol. Chem. 353, 911-920 (1972), Mastzellen, nach Mohri, K.; Reimann, H.-J. et al.: Histamin content and mastcells in human gastric and duodenal mucosa. Agents and Actions 8 (4), 372 (1978) und Prostaglandin ($PGE_2$), nach Moncada, S.; Herman, A.G. et al.: Differential formation of prostacyclin (PGX or $PGI_2$) by lysis of the arterial wall. An explanation for the anti-thrombotic properties of vascular endothelium. Thrombosis Research 11, 323 - 344 (1977).

Das Ergebnis dieser Untersuchung (Tabelle 1) zeigt, daß Iberis amara die Motilität sowohl des Magens als auch des Darmes statistisch signifikant erhöht. Außerdem ist eine cytoprotektive Wirkung zu erkennen, durch die statistisch signifikante Hemmung der mit Acetylsalicylsäure provozierten Gewebshistaminfreisetzung und der Mastzellenproliferation.

Die tonus- und motilitätsregulierende Wirkung des erfindungsgemäßen Arzneimittels nach den Ansprüchen auch STW 5-II genannt, mit der verringerten Anzahl der als Träger der Wirksamkeit in Frage kommenden Inhaltsstoffe gegenüber dem bekannten Produkt (STW 5) auf pflanzlicher Basis wurde wie folgt untersucht:

Basistonuserhöhung

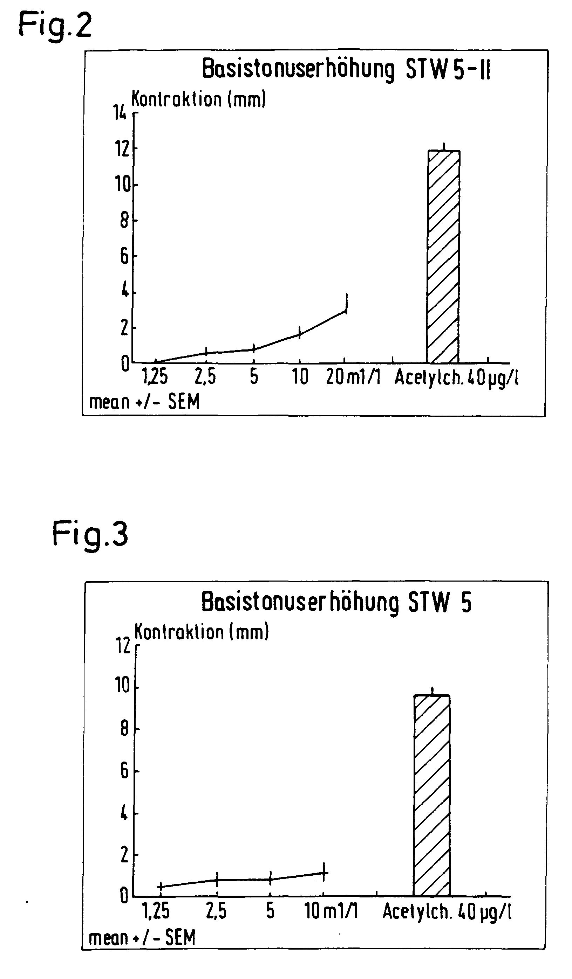

Mit dem klassischen pharmakologischen Modell des Meerschweinchen-Ileums nach Magnus wurde die basistonuserhöhende Wirkung von STW 5-II gegenüber STW 5 verifiziert. In den Tabellen 2 und 5 und den sie grafisch wiedergebenden Fig. 2 bzw. 3 sind die Ergebnisse im Vergleich mit der von Acetylcholin 40 µg/l induzierten Kontraktion dargestellt.

Das erfindungsgemäße Arzneimittel STW 5-II wirkt deutlich basistonuserhöhend bei hypotonem Darmstück und stärker als das bekannte Produkt (STW 5).

Spasmolytische Wirkung

Die spasmolytische Wirkung des Arzneimittels STW 5-II wurde auf die Histamin-induzierte Kontraktion (Spasmen) des Meerschweinchen-Ileums untersucht und mit der des bekannten alten Produktes an den gleichen Ileumstücken verglichen.

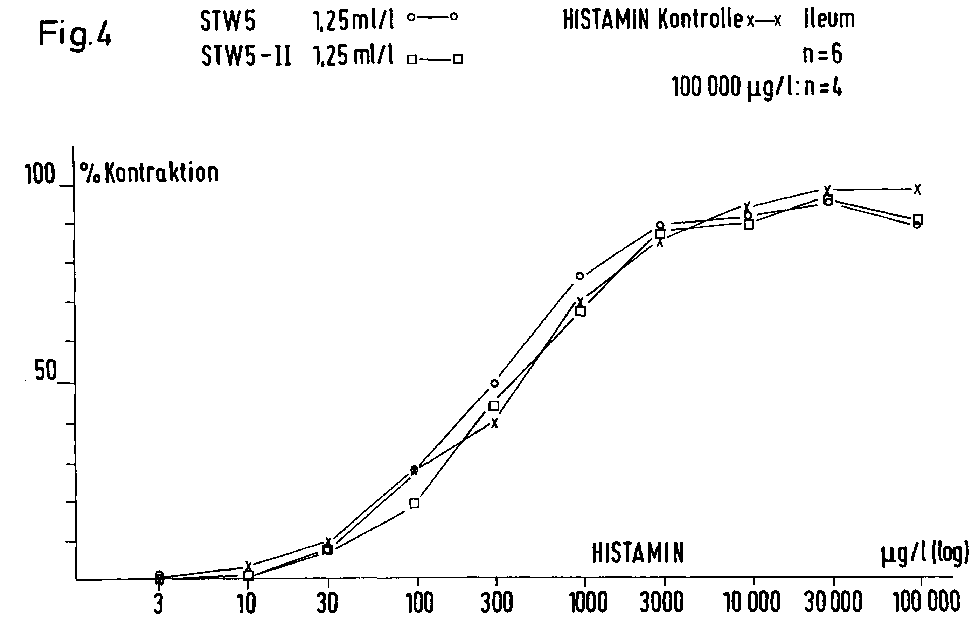

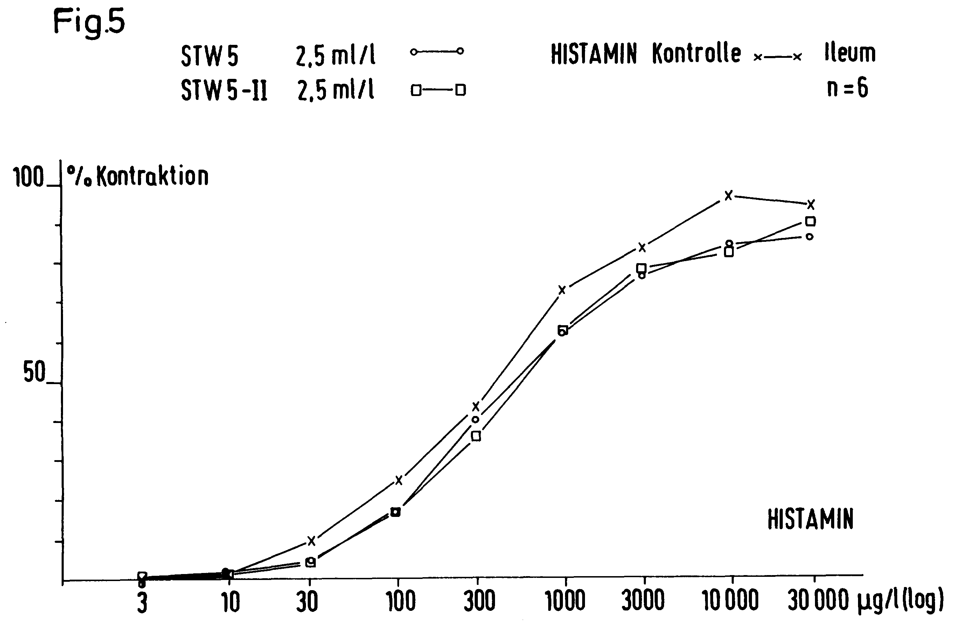

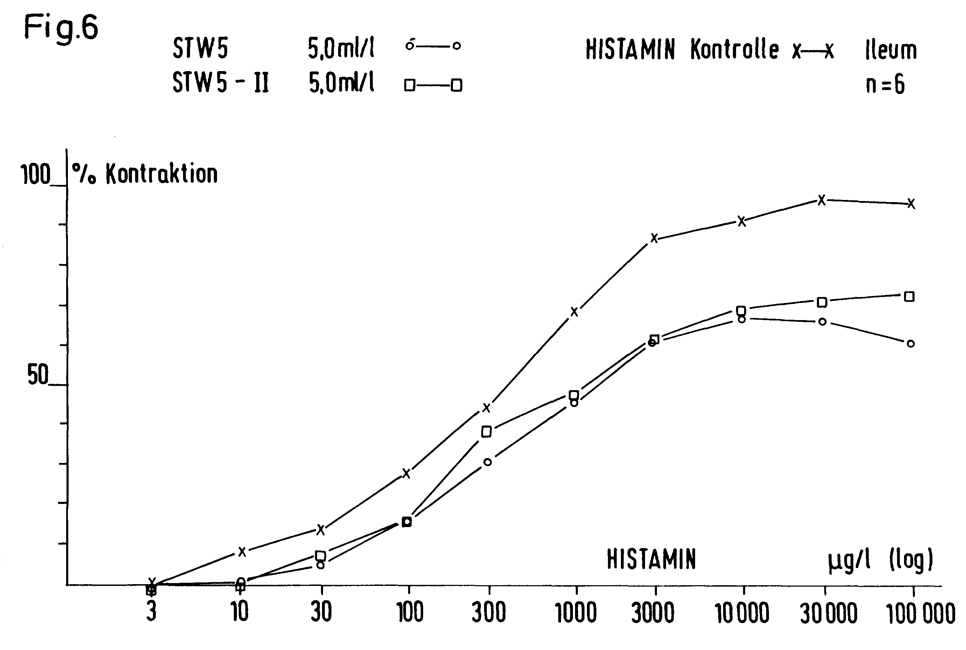

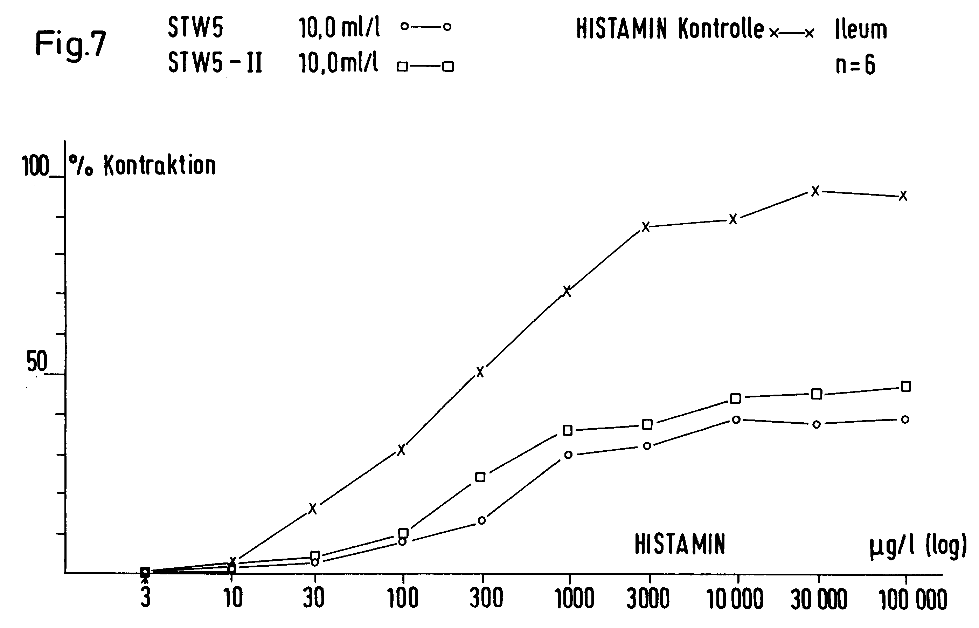

Die in den Fig. 4 bis 7 dargestellten Dosis-Wirkungskurven zeigen, daß das Arzneimittel STW 5-II dosisabhängig und statistisch signifikant spasmolytisch (krampflösend) gegen die Histamin-induzierte Kontraktion des Meerschweinchen-Ileums wirksam ist. Diese Wirksamkeit ist vergleichbar in allen Dosierungen mit der des alten Produktes, bei jedoch deutlich verringerter Anzahl von Inhaltsstoffen.

Motilität des Magen-Darm-Traktes (STW 5-II)

Die Wirkung des erfindungsgemäßen Arzneimittels nach den Ansprüchen 2 bis 9 auf die Motilität des Magen-Darm-Traktes wurde nach den vorstehend beschriebenen Methoden an Minipigs von Alaska-Hoke untersucht. Neben der Bestimmung der Motilität des Magens und des Darmes wurden pH, Azidität, Gewebshistamine, Mastzellen und Prostaglandine ($PGE_2$) vor und nach der Präparatsverabreichung gemessen.

Aus den Ergebnissen in Tabelle 4 ist zu entnehmen, daß die Arzneimittel-Kombination nach den Ansprüchen 2 bis 9 die Motilität des hypotonen Magens und des Darmes statistisch signifikant steigert. Ebenso wird die durch Acetylsalicylsäure provozierte Histaminfreisetzung und Mastzellenproliferation statistisch signifikant inhibiert, als ein Zeichen der entzündungshemmenden und zytoprotektiven Wirkung.

Ein Vergleich dieses Effektes mit der des bekannten Produktes (STW 5) (Tabelle 5) macht deutlich, daß die Wirksamkeit des Arzneimittels nach den Ansprüchen 1 bis 9 signifikant stärker einzustufen ist.

Einfluß auf die 5-Lipoxygenase-Produkte (LTB$_4$)

Sowohl die Prostaglandine (insbesondere PGE$_2$), Cycloxygenase-Produkte aus der Arachidonsäure-Kaskade als auch die Leukotriene (LTB$_4$) der Lipoxygenase-Metaboliten, zählen zu den humoralen Entzündungsmediatoren, deren Hemmung bei ulcerierenden Magen-Darm-dyspeptischen Erkrankungen sehr zu wünschen wäre.

Der Einfluß auf die 5-Lipoxygenase-Produkte (LTB$_4$) durch das Arzneimittel nach den Ansprüchen 2 bis 9 wurde an aktivierten polymorphkernigen, neutrophilen Granulozyten der Ratte in vitro nach einer Stimulierung mit Calciumionophor A23187 mittels HPLC gemessen.

Setzt man die produzierte Menge des Kontrollversuchs gleich 100 %, wurden folgende Werte in % $\overline{X} \pm$ SD für die Prüfsubstanzen erhalten:

|  |  | Hemmung |
|---|---|---|
| STW 5 | $89 \pm 19$ | 11 % |
| STW 6 | $96 \pm 7$ | 4 % |
| STW 5-II | $75 \pm 8$ | 25 % |

Die Wirkung des Arzneimittels nach den Ansprüchen 2 bis 9 bei der Hemmung der 5-Lipoxygenase-Produkte ist ebenfalls stärker als die des alten Produktes.

Entzündungshemmende Wirkung

Das Carrageenan-Ödem-Modell der Ratte stellt eine international anerkannte pharmakologische Methode dar, zur Aufdeckung von klinisch relevanten Entzündungshemmern.

Die Prüfsubstanzen wurden 1/2 h vor der Ödem-Induktion verabreicht, die Kontrolltiere erhielten das Lösungsmittel (30 % Ethanol). Die Pfotenvolumina wurden vor (O-Wert) der Ödem-Induktion sowie 1, 2, 3, 4, und 6 Stunden danach mit Plethysmometer gemessen.

Die Berechnung der entzündungshemmenden Wirkung der Prüfsubstanzen erfolgte nach der Formel

$$\% \text{ Hemmung} = 100 - ( \frac{V}{K} \cdot 100)$$

V   = Mittelwert der Differenzen zum O-Wert; Verumgruppe
K   = Mittelwert der Differenzen zum O-Wert; Kontrollgruppe

Folgende Ergebnisse wurden erzielt ( % Hemmung):

|  | 1 | 2 | 3 | 4 | 6 h |
|---|---|---|---|---|---|
|  | - nach Ödem-Setzung - | | | | |
| STW 5 5 ml/kg KG: | 24,0 | 20,6 | 25,0* | 37,5 | 38,7* |
| STW 5-II 5 ml/kg KG: | 4,0 | 14,7 | 27,5* | 35,0* | 38,7* |
| Indomethacin 4 mg/kg KG: | 24,0 | 23,5 | 35,0* | 47,5* | 61,3* |

* P $\leqq$ 0,05

Das Ergebnis zeigt, daß das Arzneimittel nach den Ansprüchen 1 bis 2 eine statistisch signifikante entzündungshemmende Wirkung in der kritischen Entzündungsphase (3 bis 6 Stunden nach der Induktion) hat, und daß diese Wirksamkeit in der Größenordnung liegt, wie die des alten Produktes und kann mit der der chemischen Substanz, Indomethacin, verglichen werden.

Das erfindungsgemäße Arzneimittel besitzt, neben der dualen Wirkung der Tonus-/Motilitätsregulierung ein deutliches entzündungshemmendes Potential durch Hemmung der Cyclo- und Lipoxygenase-Produkte, die bei dyspeptischen bzw. funktionellen und motilitätsbedingten Magen-Darmstörungen durch Schädigungen der Schleimhäute (Gastritis, Colitis, Ulcera u.a.) auftreten.

Das Arzneimittel auf pflanzlicher Basis nach den Ansprüchen 1 bis 9 vereinigt als Tonusmodulator die Vorteile eines Spasmolytikums und eines Spasmodikums (Prokinetikum) mit den Eigenschaften eines Entzündungshemmers.

Tabelle 1

| Iberis Amara (STW 6) n = 6 | | |
|---|---|---|
| | **vor** | **nach** |
| pH | $1,8 \pm 0,1$ | $1,6 \pm 0,2$ |
| Acidität | $24 \pm 4$ | $26 \pm 3$ |
| Gewebshistamin | $33 \pm 4$ | $36 \pm 4$ |
| Provokation | $34 \pm 4$ | $24 \pm 2$ ** |
| Mastzellen | --- | $62 \pm 6$ |
| Provokation | $59 \pm 3$ | $45 \pm 4$ ** |
| PGE$_2$ | $14 \pm 3$ | $13 \pm 4$ |
| Provokation | $10 \pm 2$ | $10 \pm 3$ |
| Motilität Magen | $5 \pm 3$ | $8 \pm 2$ * |
| Motilität Darm | $10 \pm 4$ | $14 \pm 3$ * |

\* = $p < 0,05$
\*\* = $p < 0,005$

## Tabelle 2

Basistonuserhöhung

Testsubstanz STW 5-11

| Konzen-tration (ml/l) | Vers. 1 | Vers. 2 | Vers. 3 | Vers. 4 | Vers. 5 | Vers. 6 |
|---|---|---|---|---|---|---|
| 1,25 | 0,0 | 0,0 | 0,0 | 0,5 | 0,0 | 0,0 |
| 2,5 | 2,0 | 0,1 | 0,4 | 0,6 | 0,1 | 0,0 |
| 5,0 | 1,0 | 0,2 | 1,1 | 1,2 | 0,0 | 0,7 |
| 10,0 | 2,0 | 0,3 | 2,1 | 3,4 | 0,2 | 1,5 |
| 20,0 | 3,0 | 0,4 | 5,4 | 6,0 | 0,4 | 2,3 |
| 40,0 | 3,0 | 0,6 | 5,1 | 6,0 | 0,3 | 1,9 |
| Acetylch. 40 µg/l | 10,1 | 14,6 | 11,7 | 14,0 | 12,7 | 8,6 |

## Mittelwerte

| Konzen-tration (ml/l) | Mittelwert | SEM | Anzahl |
|---|---|---|---|
| 1,25 | 0,08 | 0,08 | 6 |
| 2,5 | 0,53 | 0,28 | 6 |
| 5,0 | 0,70 | 0,19 | 6 |
| 10,0 | 1,58 | 0,45 | 6 |
| 20,0 | 2,92 | 0,89 | 6 |
| 40,0 | 2,82 | 0,87 | 6 |
| Acetylch. 40 µg/l | 11,95 | 0,86 | 6 |

7

## Tabelle 3

Testsubstanz STW 5

| Konzen-tration (ml/l) | Vers. 1 | Vers. 2 | Vers. 3 | Vers. 4 | Vers. 5 | Vers. 6 |
|---|---|---|---|---|---|---|
| 1,25 | 0,3 | 0,0 | 1,8 | 0,1 | 0,3 | 0,3 |
| 2,5 | 0,3 | 0,0 | 2,6 | 0,0 | 0,8 | 0,7 |
| 5,0 | 0,3 | 0,2 | 2,4 | 0,1 | 0,8 | 0,5 |
| 10,0 | 0,2 | 0,1 | 3,2 | 0,1 | 1,7 | 1,1 |
| 20,0 | 0,2 | 0,0 | 2,6 | 0,3 | 0,9 | 1,2 |
| 40,0 | 0,1 | 0,0 | 0,8 | 0,1 | 0,1 | 0,0 |
| Acetylch. 40 µg/l | 11,8 | 6,3 | 12,1 | 9,9 | 12,2 | 5,9 |

**Mittelwerte**

| Konzen-tration (ml/l) | Mittelwert | SEM | Anzahl |
|---|---|---|---|
| 1,25 | 0,47 | 0,25 | 6 |
| 2,5 | 0,73 | 0,36 | 6 |
| 5,0 | 0,72 | 0,32 | 6 |
| 10,0 | 1,07 | 0,46 | 6 |
| 20,0 | 0,87 | 0,36 | 6 |
| 40,0 | 0,18 | 0,11 | 6 |
| Acetylch. 40 µg/l | 9,70 | 1,09 | 6 |

Tabelle 4

| Combination STW 5-II n = 6 | | |
|---|---|---|
| Fol. Melissae<br>Fruct. Carvi<br>Flor. Chamomillae<br>Iberis amara<br>Fol. Menthae pip. | | |
| | **vor** | **nach** |
| pH | $1,4 \pm 0,2$ | $2,2 \pm 0,4$ |
| Acidität | $22 \pm 4$ | $29 \pm 5$ |
| Gewebshistamin | $32 \pm 5$ | $37 \pm 6$ |
| Provokation | $36 \pm 6$ | $30 \pm 7$ * |
| Mastzellen | $64 \pm 6$ | $61 \pm 3$ |
| Provokation | $68 \pm 7$ | $54 \pm 6$ * |
| PGE$_2$ | $10 \pm 4$ | $17 \pm 3$ ** |
| Provokation | $13 \pm 4$ | $12 \pm 4$ |
| Motilität Magen | $5 \pm 2$ | $11 \pm 3$ ** |
| Motilität Darm | $10 \pm 2$ | $11 \pm 3$ ** |

Tabelle 5

| Bekanntes Produkt (STW 5) | | |
|---|---|---|
| | **vor** | **nach** |
| pH | $1,4 \pm 0,3$ | $1,9 \pm 0,4$ |
| Acidität | $26 \pm 5$ | $29 \pm 4$ |
| Gewebshistamin | $37 \pm 5$ | $32 \pm 4$ |
| Provokation | $34 \pm 5$ | $24 \pm 6$ ** |
| Mastzellen | $61 \pm 4$ | $58 \pm 5$ |
| Provokation | $58 \pm 5$ | $44 \pm 5$ ** |
| PGE$_2$ | $12 \pm 3$ | $15 \pm 4$ |
| Provokation | $14 \pm 2$ | $12 \pm 3$ |
| Motilität Magen | $6 \pm 4$ | $9 \pm 4$ |
| Motilität Darm | $9 \pm 4$ | $14 \pm 5$ |

**Patentansprüche**

1. Arzneimittel auf pflanzlicher Basis mit tonisierender Wirkung auf atonische bzw. tonusgeminderte, glattmuskuläre Organe, <u>dadurch gekennzeichnet</u>, daß es Iberis amara, insbesondere als Pflanzenauszug oder dessen Inhaltsstoffe, als alleinigen Träger der Wirksamkeit enthält, wobei Chelidonii herba, Cardui mariae fructus und Angelicae radix nicht vorhanden sind.

2. Arzneimittel auf pflanzlicher Basis mit tonusmodulierender Wirkung auf tonusgeminderte oder tonusgesteigerte, glattmuskuläre Organe, dadurch gekennzeichnet, daß es Iberis amara, insbesondere als Pflanzenauszug, oder dessen Inhaltsstoffe zusammen mit einem oder mehreren weiteren Bestandteilen der Gruppe, bestehend aus

Menthae piperitae folium
Matricariae flos,
Carvi fructus
Melissae folium und
Liquiritiae radix,

vorzugsweise als Pflanzenauszüge, als alleinige Träger der Wirksamkeit enthält, wobei Chelidonii herba, Cardui mariae fructus und Angelicae radix nicht vorhanden sind.

3. Arzneimittel nach Anspruch 1 oder 2, dadurch gekennzeichnet, daß es Iberis amara totalis (Blüten, Samen, Blätter, Stengel und Wurzeln) enthält.

4. Arzneimittel nach Anspruch 1 bis 3, dadurch gekennzeichnet, daß es in Form des Pflanzenauszugs etwa

15 - 40 Vol.-% Iberis amara,
10 - 30 Vol.-% Menthae piperitae,
20 - 40 Vol.-% Matricariae,
10 - 30 Vol.-% Carvi fructus,
10 - 30 Vol.-% Melissae folium,
10 - 30 Vol.-% Liquiritiae radix

enthält.

5. Arzneimittel nach Anspruch 4, dadurch gekennzeichnet, daß es in Form des Pflanzenauszugs etwa

15 Vol.-% Iberis amara totalis,
10 Vol.-% Menthae piperitae folium,
30 Vol.-% Matricariae flos,
20 Vol.-% Carvi fructus,
15 Vol.-% Melissae folium,
10 Vol.-% Liquiritiae radix

enthält.

6. Arzneimittel nach Anspruch 1 bis 5, dadurch gekennzeichnet, daß der Pflanzenauszug ein Frischpflanzenauszug, insbesondere bei Iberis amara, ist.

7. Arzneimittel nach Anspruch 1 bis 6, dadurch gekennzeichnet, daß bei dem Frischpflanzenauszug das Verhältnis von mazerierter oder perkolierter Pflanze zum Extrakt etwa 3 bis 7 Gramm : 10 Gramm beträgt.

8. Arzneimittel nach Anspruch 1 bis 7, dadurch gekennzeichnet, daß bei dem Drogenauszug das Verhältnis von Drogen zum Extrakt etwa 1,5 bis 5 Gramm : 10 Gramm beträgt.

9. Arzneimittel nach Anspruch 1 bis 8, dadurch gekennzeichnet, daß das Extraktionsmittel Ethanol in einer Endkonzentration von 30 - 40 Vol.-%, Rest $H_2O$, ist.

**Claims**

1. Plant-based medicament having tonicising action on smooth muscular organs which are atonic or have decreased tone, characterised in that it contains Iberis amara, in particular as a plant extract or its constituents, as the sole carrier of the activity, whereby Chelidonii herba, Cardui mariae fructus and Angelicae radix are not present.

2. Plant-based medicament having tone-modulating action on smooth muscular organs which have decreased tone or increased tone, characterised in that it contains Iberis amara, in particular as a plant extract, or its constituents together with one or more other constituents from the group consisting of

Menthae piperitae folium
Matricariae flos,
Carvi fructus
Melissae folium and
Liquiritiae radix,

preferably as plant extracts, as the sole carriers of the activity, whereby Chelidonii herba, Cardui mariae fructus and Angelicae radix are not present.

3. Medicament according to Claim 1 or 2, characterised in that it contains Iberis amra totalis (flowers, seeds, leaves, stalks and roots).

4. Medicament according to Claim 1 to 3, characterised in that it contains, in the form of the plant extract, about

15 - 40% by volume of Iberis amara,
10 - 30 % by volume of Menthae piperitae,
20 - 40% by volume of Matricariae,
10 - 30% by volume of Carvi fructus,
10 - 30 % by volume of Melissae folium,
10 - 30 % by volume of Liquiritiae radix.

5. Medicament according to Claim 4, characterised in that it contains, in the form of the plant extract, about

15 % by volume of Iberis amara totalis,
10 % by volume of Menthae piperitae folium,
30 % by volume of Matricariae flos,
20% by volume of Carvi fructus,
15 % by volume of Melissae folium,
10 % by volume of Liquiritiae radix.

6. Medicament according to Claims 1 to 5, characterised in that the plant extract is a fresh plant extract, in particular Iberis amara.

7. Medicament according to Claims 1 to 6, characterised in that in the fresh plant extract the ratio of macerated or percolated plant to the extract is about 3 to 7 grams : 10 grams.

8. Medicament according to Claims 1 to 7, characterised in that in the drug extract the ratio of drugs to extract is about 1.5 to 5 grams: 10 grams.

9. Medicament according to Claims 1 to 8, characterised in that the extracting agent is ethanol in a final concentration of 30 - 40% by volume, the remainder being $H_2O$.

**Revendications**

1. Médicament à base de plante ayant un effet tonifiant sur des organes musculaires lisses atoniques, respectivement manifestant une diminution du tonus, caractérisé en ce qu'il contient Iberis amara, en particulier sous la forme d'un extrait de plante ou de ses constituants, à titre de support unique du principe actif, dans lequel Chelidonii herba, Cardui mariae fructus et Angelicae radix ne sont pas présents.

2. Médicament à base de plantes ayant un effet modulateur du tonus musculaire sur des organes musculaires lisses atoniques, respectivement manifestant une diminution ou une augmentation du tonus, caractérisé en ce qu'il contient Iberis amara, en particulier sous la forme d'un extrait de plante ou de ses constituants, conjointement avec un ou plusieurs autres composants choisis parmi le groupe constitué par

Menthae piperitae folium,
Matricariae flos,
Carvi fructus,
Melissae folium et

Liquiritiae radix,

de préférence sous la forme d'extraits de plantes, à titre de support unique du principe actif, dans lequel Chelidonii herba, Cardui mariae fructus et Angelicae radix ne sont pas présents.

3. Médicament selon la revendication 1 ou 2, caractérisé en ce qu'il contient Iberis amara totalis (des fleurs, des semences, des feuilles, des tiges et des racines).

4. Médicament selon les revendications 1 à 3, caractérisé en ce qu'il contient, sous la forme d'extraits de plantes, environ

  15-40% en volume de Iberis amara,
  10-30% en volume de Menthae piperitae,
  20-40% en volume de Matricariae,
  10-30% en volume de Carvi fructus,
  10-30% en volume de Melissae folium,
  10-30% en volume de Liquiritiae radix.

5. Médicament selon la revendication 4, caractérisé en ce qu'il contient, sous la forme d'extraits de plantes, environ

  15% en volume de Iberis amara totalis,
  10% en volume de Menthae piperitae folium,
  30% en volume de Matricariae flos,
  20% en volume de Carvi fructus,
  15% en volume de Melissae folium,
  10% en volume de Liquiritiae radix.

6. Médicament selon la revendication 5, caractérisé en ce que l'extrait de plante est un extrait de plante frais, en particulier dans le cas de Iberis amara.

7. Médicament selon les revendications 1 à 6, caractérisé en ce que, dans le cas d'un extrait de plante frais, le rapport des plantes obtenues par macération ou par percolation à l'extrait s'élève d'environ 3 à 7 grammes : 10 grammes.

8. Médicament selon les revendications 1 à 7, caractérisé en ce que, dans le cas de l'extrait sec, le rapport de la matière sèche à l'extrait s'élève d'environ 1,5 à 5 grammes : 10 grammes

9. Médicament selon les revendications 1 à 8, caractérisé en ce que l'agent d'extraction est l'éthanol en une concentration finale de 30 à 40% en volume, le reste étant $H_2O$.

# Fig.1

Einfluß von STW 6 auf den Tonus des ruhenden
isolierten Meerschweinchenileums

## Fig.2

Basistonuserhöhung STW 5-II

## Fig.3

Basistonuserhöhung STW 5

Fig.4

STW5    1,25ml/l  o——o

STW5-II  1,25 ml/l  □——□

HISTAMIN Kontrolle x—x  Ileum

n=6

100 000 µg/l : n=4

% Kontraktion

100

50

HISTAMIN

µg/l (log)

3   10   30   100   300   1000   3000   10 000   30 000   100 000

Fig.5

STW 5     2,5 ml/l     o—o          HISTAMIN Kontrolle ×—× Ileum
STW 5-II  2,5 ml/l     □—□                                 n = 6

HISTAMIN

EP 0 550 703 B1

# Fig.6

STW5      5,0ml/l    ⚬——⚬      HISTAMIN Kontrolle x——x    Ileum

STW5 – II    5,0ml/l    ☐——☐                                n=6

% Kontraktion — y-axis (0, 50, 100); x-axis: HISTAMIN µg/l (log) — 3, 10, 30, 100, 300, 1000, 3000, 10 000, 30 000, 100 000

EP 0 550 703 B1

Fig.7

STW5 10,0 ml/l ○——○
STW5 – II 10,0 ml/l □——□

HISTAMIN Kontrolle ×——× Ileum

n = 6

% Kontraktion

HISTAMIN

µg/l (log)

EP 0 550 703 B1